# EUROPEAN PATENT APPLICATION

(11) **EP 0 727 664 A2**
(43) Date of publication of application: **21.08.1996**
(21) Application number: 96101474.3
(22) Date of filing: 02.02.1996
(51) Int. Cl.: G01N 33/574

(54) **Tumor marker panel for the detection of breast cancer recurrence**

(30) Priority: 15.02.1995 US 388876
(71) Applicant: Bayer Corporation, Pittsburgh, PA 15219-2502 (US)
(72) Inventor: Allard, William Jeffrey, Poughquag, New York 12570 (US)
(74) Representative: Burkert, Frank

(57) **Abstract**

Recurrence of breast cancer is detected by the presence of abnormal tumor marker levels in a blood sample from a patient as determined by performing a panel of tumor marker assays. The tumor marker panel comprises at least two of a CA 228 assay, a CA 15-3 assay, and a TPS assay. The tumor marker panel comprising all three tumor marker assays detects recurrence in essentially 100% of all cases, and provides a median lead time of about 5.5 months.

## Description

### BACKGROUND OF THE INVENTION

The present invention relates to diagnostic methods for detecting recurrence of disease in patients that have undergone treatment for breast cancer. Further, the present invention concerns diagnostic methods for the detection of breast cancer recurrence with clinically significant lead times.

Cancer biomarkers (tumor markers) are substances which increase in concentration in the blood of patients with cancer as compared to healthy individuals. Five potential uses for cancer biomarkers have been proposed (1,2). These include screening in the asymptomatic population for occult cancer, confirmation of cancer diagnosis in symptomatic patients, prognosis in patients diagnosed with cancer by other means, monitoring the success of primary therapy, and monitoring for recurrence in patients undergoing therapy. Despite much promise following the discovery of CEA by Gold and Freedman 30 years ago (3), tumor markers are not useful tools for screening in the general population.

The exception is prostate-specific antigen (PSA) which has been recommended by the American Cancer Society and the U.S. Food and Drug Administration for early detection of prostate cancer when combined with digital rectal examination. Similarly, serum markers have shown no clear utility in prognosis at the time of presentation. Biomarkers have, however, proven useful in monitoring patients following primary therapy and in monitoring for cancer recurrence. This is especially true for the use of hCG in detection of choriocarcinomas, and for AFP in monitoring patients with testicular cancers (4,5). These markers have proven successful in these indications due to the availability of curative therapy, even in late stage choriocarcinoma and testicular cancer.

Application of cancer biomarkers to the management of patients with breast cancer has met with less success. The analytes tested for clinical utility in breast cancer include carcinoembryonic antigen (CEA), a glycoprotein expressed at low levels in colorectal and breast tissues which functions as an adhesion molecule (6); a mucinous antigen found in milk fat globules which is measured by the CA 15-3 assay (7); a fragment of cytokeratin 18 which is measured by a polyclonal antibody-based assay (tissue polypeptide antigen, TPA) and by a monoclonal antibody-based assay (tissue polypeptide specific antigen, TPS). Other assays have shown some sensitivity in the detection of breast cancer including a plethora of assays which detect the same mucin as CA 15-3 (MCA, CAM 26, CAM 29, CA 549, BCM) (7).

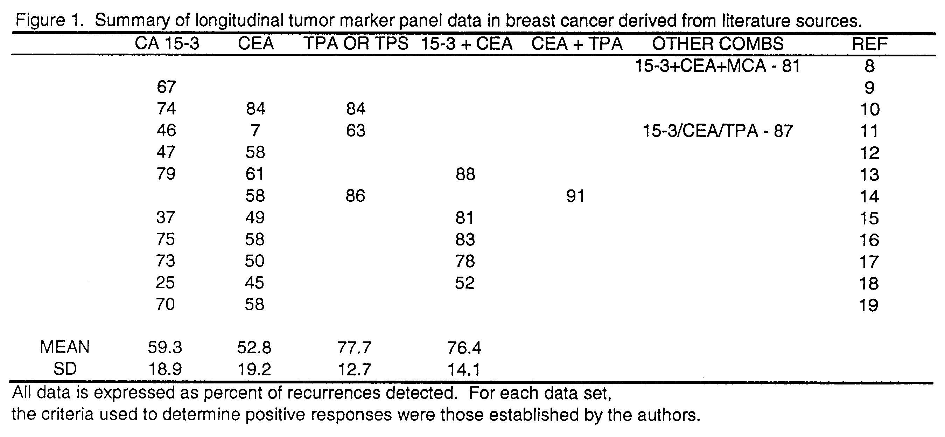

As shown in Figure 1, when patients are monitored using a single diagnostic test, breast cancer recurrence can be detected in approximately 50-70% of patients (8-19). Detection of breast cancer recurrence by biomarker monitoring is most sensitive for widespread recurrences such as metastasis to bone, viscera, and the central nervous system. Biomarkers, however, are significantly less efficient at detection of locoregional spread (9,12,17). To improve the sensitivity of early detection of breast cancer recurrence, panels of biomarkers have been proposed and tested. The results summarized in Figure 1 demonstrate that concomitant use of two markers such as CA 15-3 and CEA, increases sensitivity by approximately 5-20%. In addition, some studies applied panels of three markers and achieved a total sensitivity of 81-87%. In other studies, however, combinations of cancer biomarkers showed no advantages over the use of single markers alone. For example, Tondini *et al* (16) and Colomer *et al* (18) concluded that the combination of CA 15-3 and CEA failed to improve the results obtained with CA 15-3 alone. Similarly, Soletormos *et al* (20) showed that the addition of CEA or TPA to CA 15-3 gave no improvement in the ability to detect recurrences in breast cancer. In other studies, the increase in sensitivity for detection of breast cancer recurrence is slight (17). Taken together, these data suggest that any given combination of cancer biomarkers might improve the sensitivity for early detection of breast cancer recurrence, but whether in fact any improvement results and, if so, what increment of increased sensitivity is obtained, cannot be predicted with any accuracy based on the current state of knowledge.

### SUMMARY OF THE INVENTION

It is now been unexpectedly found that a test panel comprising at least two of a CA 228 assay, a CA 15-3 assay, and a TPS assay provides a useful means for detecting recurrence of breast cancer in a patient. When all three markers are measured, essentially 100% of all recurrences can be detected. Abnormal tumor marker blood levels can be indicated in any statistically significant manner, for example, by a determination that a tumor marker value exceeds either (i) the upper limit of normal or (ii) 110% (more usually 130%, and more preferably 150%) of the mean value of at least three assays performed on blood samples taken from the patient while showing no evidence of disease. Recurrence of breast cancer can be manifested in many different forms, such as secondary malignancy in the contralateral breast, spread of cancer to the regional lymph nodes, or spread of the tumor to more distant sites such as bone, liver, or the central nervous system. In all cases, recurrence reflects spread of the initial, primary tumor which occurred prior to removal or treatment of the primary tumor by surgery, radiation, chemotherapy, and the like. The three tumor marker panel of the present invention has also been found to provide a median lead time of approximately 5.5 months. Lead time is defined as the time between the initial rise of a tumor biomarker and the time of detection of recurrence by the physician using conventional diagnostic methods other than biomarker measurement, such as physical examination, chest X-ray, bone scan, magnetic resonance imaging (MRI), and so forth.

The present invention also provides a test kit for use in the detection of recurrence of breast cancer in a patient, comprising immunoassay reagents for performing each of a CA 228 assay, a CA 15-3 assay, and a TPS assay. Typically, such immunoassay reagents for each of said assays comprises a pair of antibody reagents specific for binding to the respective tumor marker antigen.

### BRIEF DESCRIPTION OF THE DRAWINGS

Fig. 1 is a table summarizing tumor marker data reported in the literature relating to breast cancer recurrence.

Fig. 2 is a table summarizing results of testing of healthy volunteers for CA 228, CA 15-3, and TPS assay values.

Fig. 3 is a table summarizing the incidence of detection of recurrence of disease in breast cancer patients by the tumor marker panel of the present invention.

Fig. 4 is a table summarizing the lead times to clinical recurrence as determined using the tumor marker panel of the present invention.

### DESCRIPTION OF THE PREFERRED EMBODIMENTS

As used herein, "CA 228 assay" refers to an assay for the determination of NCA 50/90. NCA (nonspecific cross-reacting antigen) was originally described as a component of normal tissue which cross-reacted with antibodies raised to CEA (Mach J-P and Pusztaszeri G (1972) *Immunochemistry* 9, 1031-1034; and von Kleist S, Chavenel G and Burtin P (1972) *Proc*. *Natl*. *Acad*. *Sci*. *USA* 69, 2492-2494). As such, NCA was considered a potential non-tumor derived interferant in assays for CEA. Molecular cloning identified a species of NCA of calculated Mᵣ 37,000 (Barnett T, Goebel SJ, Nothdurft MA and Elting JJ (1988) *Genomics* 3, 59-66; Tawargi Y, *et al* (1988) *Biochem*. *Biophys*. *Res*. *Commun*. 150, 89-96; and Neumaier M, *et al* (1988) *J*. *Biol*. *Chem*. 263, 3203-3207). This NCA has been termed NCA 50/90 since it is known to code for a mature protein of Mᵣ 50,000 which has amino acid identity with an Mᵣ 90,000 isoform. A second member of the NCA gene family was identified by molecular cloning and codes for an Mᵣ 95,000 glycoprotein termed NCA 95 (Kuroki M, *et al* (1991) *J*. *Biol*. *Chem*. 266, 11810-11817). A particular CA 228 assay method is described in the Examples below.

As used herein, "CA 15-3 assay" refers to an assay for the determination of the mucinous antigen found in fat globules as described in the literature - Gendler SJ, Spicer AP, Lalani E-N, Duhig T, Peat N, Burchell J, Pemberton L, Boshell M and Taylor-Papadimitriou J (1991) *Am Rev Resp Dis* 144:542-547; and Hilkenns J, Ligtenberg MJL, Vos HL and Litvinov SV (1992) *TIBS* 17:359-363. CA 15-3 assays can also be performed using various commercially available kits, e.g., TECHNICON IMMUNO 1® CA 15-3 Assay, Miles Diagnostics, Tarrytown, New York, USA; CENTOCOR CA 15-3 Assay, Centocor, Malvern, Pennsylvania, USA; and the CA 15-3 assay available from CIS bio international, Gif-Sur-Yvette, Cedex, France.

As used herein, "TPS assay" refers to a monoclonal antibody-based assay for tissue polypeptide specific antigen as desribed in the literature - Bjorklund B (1978) *Antibiotics Chemother* 22:16-31; and Eskelinen M, Hippelainen M, Salmela E, Paajanen H, Alhava E and Syrjanen K (1992) *Anticancer Res* 12:2033-2036. Commercial test kits are also available, e.g, from BEKI Diagnostics, Bromma, Sweden.

The present invention is not limited to any particular method for performing the individual tumor marker assays. Essentially any quantitative method may be employed in the measurement of blood (e.g., serum or plasma) tumor marker levels. Typically, however, immunoassays will be employed in which the tumor marker antigen of interest is determined based upon specific binding of an antibody reagent. Conventional polyclonal or monoclonal antibodies can be employed in immunoassay formats and methods that are well known in the art. Typically, such measurement will be performed by two-site immunometric, or sandwich, immunoassay using two antibody reagents; one of which recognizes the tumor marker to the exclusion of other potentially related substances, while the other is capable of binding specifically or nonspecifically with the tumor marker. It will be generally preferred in sandwich immunoassay methods that at least one monoclonal antibody be employed. Assay format and methods for the preparation of the required antibody reagents can be selected by the skilled worker in the field. Suitable antibody reagents can be labeled, e.g., enzyme-labeled, or immobilized, e.g., coated onto a microtiter plate, bound to plastic or magnetic beads or particles, and can be comprised of whole immunoglobulins, e.g., IgG or IgM, or fragments, e.g., Fab, Fab', and F(ab')₂ fragments, or aggregates thereof.

As described above, commercial kits are available for performing CA 15-3 assays and TPS assays. Alternatively, immunoassay reagents for performing CA 15-3 assays and TPS assays, and for performing CA 228 assays, can be prepared in conventional manners following the teachings in the literature. Antibody reagent for a particular tumor marker can be prepared by immunization of a host animal with a suitable immunogen such as a tumor marker-containing immunogen mixture, e.g., a purified extract of tumor cells; tumor marker-expressing transfectant cell lines (see European Patent Publication 346, 702 in regard to NCA 50/90); an immunogen conjugate comprising a synthetically prepared peptide coupled to a conventional immunogenic carrier molecule, where the peptide has an amino acid sequence encompassing an epitope of the tumor marker; and the like as will be understood in the art.

Antibody reagents comprising monoclonal antibodies will be generally preferred. Particularly preferred NCA 50/90 specific monoclonal antibodies useful in a CA 228 assay are those which bind to substantially the same epitope as that produced by the hybridoma that has been deposited with the American Type Culture Collection (ATCC), Rockville, Maryland, USA, and identified as ATCC HB 11204. It will be understood that a number of standard methods can be used in order to determine whether a particular monoclonal antibody binds to substantially the same epitope as the above-mentioned antibody whose hybridomas have been deposited with the ATCC. A particularly useful method is competitive binding, wherein the ability of the antibody of interest to bind to NCA 50/90 in the presence of the reference antibody is measured. Substantial inability of both antibodies to bind simultaneously indicates that substantially the same epitope is involved.

After performing the three tumor marker assays on the patient's blood sample (e.g., serum or plasma), a determination is then made whether any one or more of the tumor marker levels are abnormally elevated. Abnormal elevation in one or more of the tumor marker levels indicates a high risk of recurrence. Abnormal tumor marker levels can be defined in any statistically significant manner. In general, tumor markers are considered elevated when the measured value exceeds the upper limit of normal. An upper limit of normal is typically defined as the mean plus twice the standard deviation for a statistically significant number of healthy individuals who are free of overt cancer. Another method for determining abnormal elevation in tumor marker values is to establish a longitudinal mean value when the patient shows no evidence of disease following treatment for the primary cancer. For example, a patient may have surgery to remove a breast tumor, followed by some combination of radiation, chemotherapy, and hormonal therapy. Some time after surgery, no evidence of breast cancer can be found clinically (the NED period - no evidence of disease). Blood samples taken during this period may be used to establish a normal value for that patient. Subsequent elevations in the value of the cancer marker may signal a recurrence of the cancer. Typical increases which have been considered significant are in the range of 10% (or 110% of the mean value of samples taken during NED) up to 30%. Another approach to defining abnormal tumor marker levels involves determination of the variability associated with the assay, and the variability in the blood concentration of the tumor marker as measured over time in healthy individuals. These two components of assay variability can be used to determine the minimum increase considered to signal disease recurrence (Tondini C, Hayes DF and Kufe DW (1989) *Hematol*/*Oncol Clin North America* 3:653-674). Other statistical methods of establishing cut-off values for abnormal tumor marker levels will be evident to the worker in the field.

The present invention will now be illustrated, but is not intended to be limited by, the following examples.

### EXAMPLES

### MATERIALS AND METHODS

Patient Samples - Serum samples were obtained from healthy female volunteers attending the Gynecology and Obstetrics Clinic at Hartford Hospital, Hartford, Connecticut. All donors attended the clinic for a yearly physical examination and were patients of the practice. All healthy volunteers were believed to be free of cancer, but comprehensive testing to rule out possible occult malignancy was not done. Samples were collected under a protocol approved by the Institutional Review Board. Similarly, sera from breast cancer patients were obtained as discarded samples from patients attending the Duke University Comprehensive Cancer Center. All samples were tested retrospectively.
For patients with breast cancer, clinical status was determined by the attending physician and classified as no evidence of disease (NED) or clinical evidence of disease (CED). CED patients were further classified as having stable or progressive malignancies. The determination of recurrence was made by attending physicians based on results of X-rays, bone scans, CT scans, and other diagnostic modalities. A total of 21 patients developed recurrent disease during the course of the study. Of these 21 patients with breast cancer recurrence, 18 had primary recurrences and 3 had secondary recurrence. All recurrences were preceded by a period where at least three samples were obtained when the patient was classified with no evidence of disease (n=19) or with stable disease (n=2).
Serum Biomarker Testing - The CA 228 Assay is a two-site sandwich immunoassay which quantitatively measures nonspecific cross-reacting antigen 50/90 (NCA 50/90) in serum and plasma. The assay uses a monoclonal antibody specific for NCA 50/90 for the capture phase, and a cross-reactive polyclonal antibody to CEA as the detector phase.
96-well ELISA plates (Immulon 4, Dynatech, Laboratories, Chantilly, Virginia, USA) were coated with 50 µl of 228.2 monoclonal antibody (ATCC HB11204, *supra*) at 5 µg/ml in 0.1 M NaHCO₃, pH 9.0 and incubated overnight at 4°C. Wells were emptied and unreacted sites on the plates were quenched by the addition of 200 µl of 25 mM Tris, pH 7.5/150 mM NaCl/0.05% Tween 20/0.1% NaN₃ (TBST) with 5% bovine albumin (BSA, fraction V, Sigma, Catalog No. A-7030) followed by a 1 hr incubation at 37°C. Wells were washed 6 times with TBST, and 50 µl of either NCA 50/90 calibrators (below) or patient sample diluted 1:51 in 25 mM HEPES, pH 7.0/250 mM NaCl/100 µg/ml mouse IgG/2.5 % BSA/50 µg/ml gentamycin/0.1 % (w/v) NaN₃ (sample diluent) was added to each well and the plates were incubated for 2 hr at 37°C. After washing 6X, a 50 µl volume of a 2.5 µg/ml solution of goat anti-CEA-alkaline phosphatase (below) in 50 mM HEPES, pH 7.0/150 mM NaCl/1 mM MgCl₆·H₂O/0.1 mM ZnCl₂/5 % BSA/50 µg/ml gentamycin/0.1 % NaN₃ (conjugate diluent) was added to all wells. After a 1 hr incubation at 37°C, the plates were washed 12X with TBST and incubated with 50 µl of p-nitrophenyl phosphate in DEA substrate buffer (Pierce, Rockford, Illinois, USA) for 30 min. The reaction was stopped with 100 µl 1 N NaOH and absorbance at 405 nm minus absorbance at 490 nm determined using a microplate reader (Thermoₘₐₓ, Molecular Devices Corp., Sunnyvale, California, USA). The amount of NCA 50/90 was determined for each test sample by comparison with the calibrator standard curve using a nonlinear spline curve fit program.
NCA calibrators comprised recombinant NCA 50/90 in concentrations from 0-100 ng/ml in 25 mM TRIS, pH 7.5, 150 mM NaCl, 0.05% (v/v) Tween 20, 5% (w/v) BSA, 50 µg/ml gentamycin, 0.1% NaN₃. Recombinant NCA 50/90 was prepared as follows. A cDNA corresponding to NCA 50/90 was derived from the breast tumor cell line BT-20 as described previously (Barnett T, Goebel SJ, Nothdurft MA and Elting JJ (1988) *Genomics* 3:59-66). The coding region for the NCA 50/90 gene was modified by the elimination of the C-terminal hydrophobic region which signals replacement by a phosphoinositol glycan linkage, and the addition of a stretch of six histidine residues, also at the carboxyl terminus of the molecule (Drake L and Barnett T (1992) *Biotechniques* 12:645-649). This construct was cloned into pVL1393 by PCR and expressed using recombinant baculovirus phage to infect Spodoptera frugiperda (Sf9) cells. NCA 50/90 was affinity purified from Sf9 supernatant fluids using a zinc-imidoacetate-Sepharose® column as described (Drake and Barnett, supra). The concentration of NCA 50/90 was determined by the BCA protein assay (Pierce, Cat. No. 23225G). Recombinant NCA 50/90 is also produced by cell line CRL 9732 on deposit with the American Type Culture Collection, Rockville, Maryland, USA.
Goat anti-CEA-alkaline phosphatase was prepared as follows. Alkaline phosphatase was conjugated to a polyclonal anti-CEA antibody preparation by the introduction of free sulfhydryl groups on the antibodies using 2-iminothiolane, and the introduction of free maleimide groups on alkaline phosphatase using the heterobifunctional crosslinker sulfo-SMCC. After purification of the reactive species, the two proteins react spontaneously to form a thioether linkage. Specifically, a 12 mg/ml solution of alkaline phosphatase (from calf intestine, Biozyme International, San Diego, California, USA) in 25 mM Na₂HPO₄, pH 7.0, 0.05 mM ZnCl₂ was activated with Sulfo-SMCC (Pierce) at 4.36 mg/ml for 25 min at room temperature. Polyclonal IgG to CEA (affinity purified from goat, BiosPacific, Emeryville, California, USA) was suspended at 10 mg/ml and activated in 1.38 mg/ml 2-iminothiolane in 100 mM triethanolamine, 100 mM NaCl, 1 mM ethylenediaminetetraacetic acid (EDTA) and reacted for 10 min at room temperature. The reactive alkaline phosphatase and antibody molecules were separated from unreacted sulfo-SMCC and 2-iminothiolane by passage over Sephadex G-50 columns. The alkaline phosphatase was concentrated to 9 mg/ml, the antibody was concentrated to 5 mg/ml, and the reactive species were mixed at 2.5 mg/ml for the IgG and at 4.4 mg/ml for the alkaline phosphatase. This gave a 2:1 molar ratio of alkaline phosphatase to IgG. After a 3 hr incubation at room temperature followed by an overnight flush with N₂ at 4°C, the conjugate was filtered through a 0.2 µm cellulose acetate membrane and concentrated to approximately 20 mg/ml total protein for passage over a Superose 6 column. Fractions were monitored by absorbance at 280 nm, pooled, and brought to 5 mg/ml BSA, 0.1% NaN₃.
The TPS assay is an EIA method which measures fragments of cytokeratin 18 in serum. The assay was obtained from BEKI Diagnostics (Bromma, Sweden) and used according to the manufacturer's instructions. The CA 15-3 assay measures an epitope exposed on the epithelial mucin, episialin, a product of the MUC-1 gene. The assay is a 2-site radiometric immunoassay and was carried out according to the manufacturer's protocol (Centocor, Malvern, Pennsylvania, USA).

### RESULTS

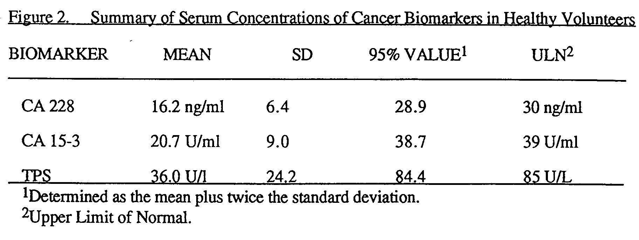

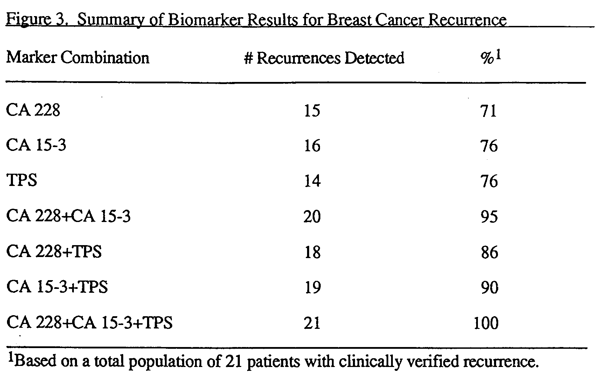

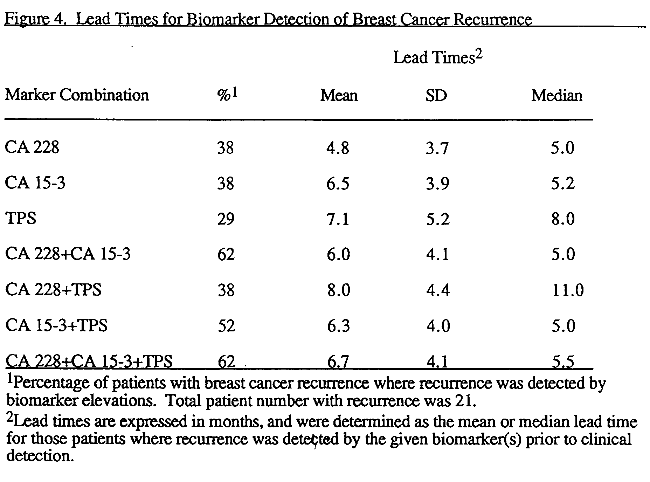

Upper Limit of Normal - A total of 208 serum samples from healthy volunteers were tested in the CA 228, CA 15-3 and TPS assays. A value for the 95th percentile was calculated assuming a normal distribution by using the mean plus twice the standard deviation. The calculated Upper Limit of Normal (ULN) for each of the three involved assays is shown in Figure 2. The Upper Limit of Normal found for the CA 15-3 Assay of 39 U/ml is somewhat higher than the 28.8 U/ml determined by the manufacturer. Because the population of healthy volunteers participating in this study was a large and well characterized cohort, 39 U/ml was used as an upper limit of normal for the CA 15-3 assay. This value is similar to values previously reported for the upper limit of normal for the CA 15-3 assay of 25 U/ml (12,17,21), 32 U/ml (11), 33 U/ml (13), 37 U/ml (9), and 40 U/ml (9,15).
Sensitivity of Biomarker Panel for Detection of Breast Cancer Recurrence - An algorithm for biomarker detection of breast cancer recurrence was established based on the upper limits of normal as described above, and on previously described methods. Biomarkers were considered to signal recurrence if one of two conditions were met. First, when the biomarker values rose above the upper limit of normal (95th percentile value), the increased value was considered positive. Second, for each patient with recurrence, a minimum of 3 measurements taken while the patient showed no evidence of disease was used to calculate the mean biomarker value in the NED state. An increase in subsequent measurements of ≧ 50% over the mean NED value was also considered diagnostic for recurrence. This value of a 50% increase agrees well with previous studies where increases of 10-30% were used as an indication of recurrent breast cancer (11,14,16).
Results shown in Figure 3 demonstrate that each of the 3 biomarkers evaluated in this study detect approximately 75% of breast cancer recurrences. When 2 of these biomarkers are used together in a panel, the sensitivity of detection of recurrence is increased by 15-20%. A further increase in sensitivity to 100% of all patients with recurrent breast cancer was seen with a combination of all three diagnostic tests.
Lead Times for Early Detection of Breast Cancer Recurrence - Detection of breast cancer recurrence is expected to have clinical utility only if the recurrence is detected in an earlier time frame than that achievable with conventional diagnostic modalities. The ability of the biomarker panel of the present invention was, therefore, examined to detect breast cancer recurrence prior to detection by conventional means. As shown in Figure 4, individual biomarkers detected recurrence with a lead time in only approximately 1/3 of cases. Some combinations of biomarkers, however, increased the proportion of cases with lead times by as much as 200%. The most efficient tumor marker combination is clearly CA 228 and CA 15-3. The addition of TPS to this panel does not improve the sensitivity of recurrence detection with lead times, but it does improve the mean lead time slightly. This improvement in lead time would be predicted based on the improved lead time achieved with TPS alone (7.1 months) as compared with CA 228 (4.8 months) or CA 15-3 (6.5 months).
Summary - The results of the studies described here demonstrate that marker panels improve diagnostic utility of cancer biomarkers in two ways. First, the combination of CA 228 with CA 15-3 and TPS detected breast cancer recurrence in 100% of the patients studied. This was unexpected in light of previous results which would have predicted an overall sensitivity of approximately 75-85% for a panel of 2-3 tumor markers (Table 1). In addition, the marker panel of the present invention provided lead times of 5-6 months in approximately 60% of patients, which was twice the proportion of patients as compared with any of the markers when used singly.

The present invention has been particularly described and exemplified above. Clearly, many other variations and modifications of the invention can be made without departing from the spirit and scope hereof.

### BIBLIOGRAPHY

1. Kvinnsland S. (1991) Serum tumor markers in clinical practice. Some general aspects. *Scan J Clin Lab Invest (Suppl)* 206:6-11.
2. Bates S E. (1991) Clinical applications of serum tumor markers. *Ann Intern Med* 115:623-638.
3. Gold P and Freedman S O. (1965) Demonstration of tumor specific antigens in human colonic carcinomata by immunological tolerance and absorbtion techniques. *J Exp Med*. 121:439-462.
4. Neville A M. (1976) Biochemical monitoring of cancer: A review. *Ann Clin Biochem*. 13:283-305.
5. Sell S. (1990) Cancer markers of the 1990s. Comparison of the new generation of markers defined by monoclonal antibodies and oncogene probes to prototypic markers. *Clin Lab Med*. 10:1-37.
6. Thompson J A, Grunert F, and Zimmerman W. (1991) Carcinoembryonic antigen gene family: Molecular biology and clinical perspectives. *J Clin Lab Anal* 5:344-366.
7. Stenman U-H and Heikkinen R. (1991) Serum markers for breast cancer. Scan *J Clin Lab Invest* 206(Suppl):52-59.
8. Repetto L, Onetto M, Gardin G, et al. (1993) Serum CEA, CA 15-3, and MCA in breast cancer patients: a clinical evaluation. *Cancer Detect Prevent* 17:411-415.
9. Bombardieri E, Pizzichetta M, Veronisi P et al. (1992) CA 15-3 determination in patients with breast cancer: clinical utility for the detection of distant metastases. *Eur J Cancer* 29A: 144-146.
10. Ng J S, Sturgeon C M, Seth J, Paterson G M, Roulston J E, Leonard R C. (1993) Serological markers for metastatic breast cancer. *Dis Markers* 11:217-223.
11. Nicolini A, Colombini C, Luciani L, Carpi A, Giuliani L. (1991) Evaluation of serum CA 15-3 determination with CEA and TPA in the post-operative follow-up of breast cancer patients. *Br*. *J*. *Cancer* 64: 154-158.
12. O'Dwyer P J, Duffy M J, O'Sullivan F, McDermott E, Losty P, O'Higgins N J. (1990) CEA and CA 15-3 in primary and recurrent breast cancer. *World J Surg* 14:562-566.
13. Tsavaris N, Vonorta K, Sarafidou M et al. (1992) Comparison of tumor markers CEA, CA 125, CA 15-3 and prolactin levels in patients with advanced breast cancer. *Diagn Oncol* 2:211-219.
14. Nicolini A, Carpi A, Di Marco G, Giuliani L, Giordani R and Palla S. (1989) A rational postoperative follow-up with carcinoembryonic antigen, tissue polypeptide antigen, and urinary hydroxyproline in breast cancer patients. *Cancer* 63:2037-2046.
15. Jager W. (1993) The early detection of disseminated (metastasized) breast cancer by serial tumour marker measurements. *Eur J Cancer Prev* 2(Suppl 3):133-139.
16. Tondini C, Hayes D F, Gelman R, Henderson I C and Kufe DW. (1988) Comparison of CA 15-3 and carcinoembryonic antigen in monitoring the clinical course of patients with metastatic breast cancer. *Cancer Res* 48:4107-4112.
17. Safi F, Kohler I, Rottinger E and Beger H-G. (1991) The value of the tumor marker CA 15-3 in diagnosing and monitoring breast cancer. A comparative study with carcinoembryonic antigen. *Cancer* 68:574-582.
18. Colomer R, Ruibal A, Genolla J, Rubio D, Del Campo J M, Bodi R and Salvador L. (1989) Circulating CA 15-3 levels in the postsurgical follow-up of breast cancer patients and in non-malignant diseases. *Breast Cancer Res Treat* 13:123-133.
19. Dnistrian A M, Schwartz M K, Greenberg E J, Smith C A, Schwartz D C. (1991) Ca 15-3 and carcinoembryonic antigen in the clinical evaluation of breast cancer. *Clin Chim Acta* 200:81-94.
20. Soletormos G, Nielsen D, Schioler V, Skovsgaard T, Winkel P, Mouridsen H T and Dombernowsky P. (1993) A novel method for monitoring high risk breast cancer with tumor markers: CA 15-3 compared to CEA and TPA. *Ann Oncol* 4:861-869.
21. Hayes D F, Zurawski V R, and Kufe D W. (1986) Comparison of circulating CA 15-3 and carcinoembryonic antigen levels in patients with breast cancer. J Clin *Oncol* 4:1542-1550.

## Claims

1. A method for the detection of recurrence of breast cancer in a patient, comprising the steps of:
(a) performing at least two tumor marker assays on a blood sample obtained from such patient, such two assays being selected from a CA 228 assay, a CA 15-3 assay, and a TPS assay, and
(b) determining if any of the measured tumor marker values show abnormal elevation.

2. The method of claim 1 wherein all three tumor marker assays are performed on said patient sample.

3. The method of claim 1 wherein a CA 228 assay and a CA 15-3 assay are performed on said patient sample.

4. The method of any one of claims 1-3 wherein step (b) is performed by determining if any of the measured tumor marker values exceeds either:
(i) the upper limit of normal, or
(ii) 110% of the mean value of at least three assays performed on blood samples taken from the patient while showing no evidence of disease.

5. The method of any one of claims 1-3 wherein step (b) is performed by determining if any of the measured tumor marker values exceeds either:
(i) the upper limit of normal, or
(ii) 130% of the mean value of at least three assays performed on blood samples taken from the patient while showing no evidence of disease.

6. The method of any one of claims 1-3 wherein step (b) is performed by determining if any of the measured tumor marker values exceeds either:
(i) the upper limit of normal, or
(ii) 150% of the mean value of at least three assays performed on blood samples taken from the patient while showing no evidence of disease.

7. A test kit for use in the detection of recurrence of breast cancer in a patient, comprising immunoassay reagents for performing at least two of a CA 228 assay, a CA 15-3 assay, and a TPS assay.

8. The test kit of claim 7 comprising immunoassay reagents for perfoming all three tumor marker assays.

9. The test kit of claim 7 comprising immunoassay reagents for performing a CA 228 assay and a CA 15-3 assay.

10. The test kit of any one of claims 7-9 wherein the immunoassay reagents for each of said assays comprises a pair of antibody reagents specific for binding to the respective tumor marker antigen.
